(19) **Europäisches Patentamt**

**European Patent Office**

**Office européen des brevets**

(11) **EP 1 629 766 A1**

(12) **EUROPEAN PATENT APPLICATION**

(43) Date of publication:
**01.03.2006 Bulletin 2006/09**

(51) Int Cl.:
***A61B 5/00*** *(2006.01)*

(21) Application number: **04027098.5**

(22) Date of filing: **15.11.2004**

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: **25.08.2004 JP 2004245646**

(71) Applicant: **Hitachi, Ltd.**
**Tokyo (JP)**

(72) Inventors:
- **Cho, Ok-Kyung,**
  **Hitachi, Ltd.,**
  **Intel. Prop. Group**
  **Chiyoda-ku**
  **Tokyo 100-8220 (JP)**

- **Kim, Yoon-Ok,**
  **Hitachi, Ltd.,**
  **Intel. Prop. Group**
  **Chiyoda-ku**
  **Tokyo 100-8220 (JP)**
- **Ouchi, Katsumi,**
  **Hitachi, Ltd.,**
  **Intel. Prop. Group**
  **Chiyoda-ku**
  **Tokyo 100-8220 (JP)**
- **Mitsumaki, Hiroshi,**
  **Hitachi Ltd**
  **Intel. Prop. Group**
  **Chiyoda-ku**
  **Tokyo 100-8220 (JP)**

(74) Representative: **Strehl Schübel-Hopf & Partner**
**Maximilianstrasse 54**
**80538 München (DE)**

(54) **Blood sugar level measuring apparatus**

(57)     An apparatus for accurately determining the blood hemoglobin concentration without taking a blood sample. The apparatus comprises an ambient temperature measuring portion for measuring ambient temperature, a radiation heat detector for measuring radiation heat from a body surface, light sources for irradiating the body surface with light of the wavelengths of at least 810 nm and 950 nm, photodetectors for detecting reflected light from the body surface, a converting portion in which measurement values relating to the blood hemoglobin concentration measured using a blood sample that is separately collected, said converting portion converting the stored measurement value, the individual outputs of the ambient temperature measuring portion, the radiation heat detector, and the photodetector, into a plurality of parameters, and a processing portion in which relationships between the parameters and blood sugar levels are stored, said calculating portion calculating a blood sugar level by applying the parameters to the stored relationships.

FIG. 2

EP 1 629 766 A1

**Description**

CLAIM OF PRIORITY

The present application claims priority from Japanese application JP 2004-245646 filed on August 25, 2004, the content of which is hereby incorporated by reference into this application.

CO-PENDING APPLICATION

US Patent application No.10/620,689 is a co-pending application of this application. The content of which is incorporated herein by reference.

BACKGROUND OF THE INVENTION

Field of the Invention

The present invention relates to a non-invasive blood sugar level measuring apparatus for measuring the glucose concentration in a living body without blood sampling.

Description of Related Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on the knowledge gained from such researches, Cho *et al.* suggest a method and apparatus for determining blood glucose concentration by temperature measurement without requiring the collection of a blood sample (Patent Documents 1 and 2).
Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. A representative value of the second-order differentiated value of absorbance is then calculated, and the representative value is corrected in accordance with the difference between the living body temperature and a predetermined reference temperature. A blood sugar concentration corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and transmitted light following the irradiation of the sample is taken, and then a glucose concentration is calculated in accordance with a linear expression of the logarithm of the output ratio and the living body temperature.

(Non-Patent Document 1) Diabete & Metabolisme, "Facial and sublingual temperature changes following intravenous glucose injection in diabetics" by R.M. Hilson and T.D.R. Hockaday, 1982, 8, 15-19)
(Non-Patent Document 2) Can. J. Physiol. Pharmacol., "Diabetes mellitus and thermoregulation", by A.R. Scott, T. Bennett, I.A. MacDonald, 1987, 65, 1365-1376
[Patent Document 1] U.S. Patent No. 5,924,996
[Patent Document 2] U.S. Patent No. 5,795,305
[Patent Document 3] JP Patent Publication (Kokai) No. 2000-258343 A
[Patent Document 4] JP Patent Publication (Kokai) No. 10-33512 A (1998)
[Patent Document 5] JP Patent Publication (Kokai) No. 10-108857 A (1998)

SUMMARY OF THE INVENTION

Glucose (blood sugar) in blood is used for glucose oxidation reaction in cells to produce necessary energy for the maintenance of living bodies. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also fluctuates due to factors other than blood glucose concentration. While methods have been proposed to determine blood glucose concentration by temperature measurement without blood sampling, they could hardly be considered sufficiently accurate.

It is an object of the invention to provide an apparatus for determining blood glucose concentrations with high accuracy without blood sampling.

Blood sugar is delivered to the cells throughout the human body via blood vessel systems, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the,cells via blood. The volume of oxygen supply is determined by the blood hemoglobin concentration, the hemoglobin oxygen saturation, and the volume of blood flow. On the other hand, the heat produced in the body by glucose oxidation is dissipated from the body by thermal convection, heat radiation, conduction, and so on. On the assumption that the body temperature is determined by the balance between the amount of energy produced in the body by glucose burning, namely heat production, and heat dissipation such as mentioned above, the inventors set up the following model:

(1) The amount of heat production and the amount of heat dissipation are considered equal.
(2) The amount of heat production is a function of the blood glucose concentration and the volume of oxygen supply.
(3) The volume of oxygen supply is determined by the blood hemoglobin concentration, the blood hemoglobin oxygen saturation, and the volume of blood flow in the capillary blood vessels.
(4) The amount of heat dissipation is mainly determined by heat convection and heat radiation.

According to this model, we achieved the present invention after realizing that blood sugar levels can be accurately determined on the basis of the results of measuring the temperature of the body surface and parameters relating to the blood oxygen concentration and the blood flow volume. The parameters can be measured, e.g., from a part of the human body, such as the fingertip. The parameters relating to convection and radiation can be determined by measuring the temperature on the fingertip. The parameters relating to the blood hemoglobin concentration and the blood hemoglobin oxygen saturation can be determined by spectroscopically measuring blood hemoglobin and then finding the ratio between hemoglobin bound with oxygen and hemoglobin not bound with oxygen. The parameter relating to the volume of blood flow can be determined on the basis of the ratio between the blood hemoglobin concentration inside tissue and the blood hemoglobin concentration in a blood sample.

In one aspect, the invention provides a blood sugar level measuring apparatus comprising: a heat amount measuring portion for measuring a temperature deriving from a body surface in order to obtain information used for the calculation of the amount of heat transfer due to convection and the amount of heat transfer due to radiation which are related to the dissipation of heat from said body surface; an optical measuring portion for irradiating said body surface with light in order to obtain measurement values related to the blood hemoglobin concentration and the hemoglobin oxygen saturation inside tissue; a memory portion for storing a measurement value relating to the blood hemoglobin concentration measured using a blood sample that is separately collected, and for storing relationships between parameters corresponding to said temperature, said blood hemoglobin concentration and said hemoglobin oxygen saturation inside said tissue, the volume of blood flow inside tissue, and blood sugar levels; a calculation portion for converting a plurality of measurement values entered via said heat amount measuring portion and said optical measuring portion, into said parameters individually, and for calculating a blood sugar level by applying said parameters to said relationships stored in said memory portion; and a display portion for displaying the result of calculation in said calculation portion, wherein the parameter corresponding to said blood flow volume inside tissue is generated on the basis of a ratio between the blood hemoglobin concentration inside tissue and the blood hemoglobin concentration measured using said blood sample.

In another aspect, the invention provides a blood sugar level measuring apparatus comprising: an ambient temperature measuring portion for measuring ambient temperature; a radiation heat detector for measuring radiation heat from a body surface; a light source for irradiating said body surface with at least light of a first wavelength and light of a second wavelength; a photodetector for detecting reflected light that is produced as said light from said light source is reflected by said body surface; a memory portion for storing a measurement value relating to the blood hemoglobin concentration that is measured using a blood sample that is separately collected; a calculating portion including a converting portion for converting said stored measurement value and the individual outputs of said ambient temperature measuring portion, said radiation heat detector, and said photodetector into a plurality of parameters, and a processing portion in which relationships between said parameters and blood sugar levels are stored, said processing portion calculating a blood sugar level by applying said parameters to said relationships; and a display portion for displaying a result outputted from said calculating portion. Preferably, the first and the second wavelengths are substantially 810 nm and 950 nm, respectively. Light of the wavelengths of substantially 810 nm and 950 nm means light with peak components at 810 nm and 950 nm, respectively. Thus, the light may include light with wavelengths of± 10 nm, and it may also include the generally expected range of error in cases where light-emitting diodes are used as light sources.

In accordance with the invention, it becomes possible to determine blood sugar levels in a non-invasive measurement with similar levels of accuracy to the conventional invasive method.

In accordance with the invention, an apparatus and method for non-invasively measuring blood sugar levels with high accuracy can be provided.

BRIEF DESCRIPTION OF THE DRAWINGS

Fig. 1 shows the relationships between measurement values provided by various sensors and the parameters derived therefrom.

Fig. 2 shows a top plan view of a non-invasive blood sugar level measuring apparatus according to the invention.

Fig. 3 shows a block diagram of the circuitry inside the apparatus.

Fig. 4 shows a measuring portion in detail.

Fig. 5 shows the flow of software and hardware operations during the operation of measuring the glucose concentration.

Fig. 6 shows the flow of software and hardware operations during the operation of measuring the glucose concentration.

Fig. 7 shows the flow of software and hardware operations during the operation of measuring the glucose concentration.

Fig. 8 shows the flow of software and hardware operations during the operation of inputting subject information.

Fig. 9 shows the flow of software and hardware operations during the operation of inputting the blood hemoglobin concentration.

Fig. 10 shows a conceptual chart illustrating the flow of data processing in the apparatus.

Fig. 11 shows a chart plotting the glucose concentration values calculated according to the present invention and the glucose concentration values measured by the enzymatic electrode method.

DETAILED DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature according to the Newton's law of cooling. The amount of heat dissipation due to radiation, which is another main cause of dissipation, is proportional to the fourth power of the body-surface temperature according to the Stefan-Boltzmann law. Thus, it can be seen that the amount of heat dissipation from the human body is related to the room temperature and the body-surface temperature. On the other hand, the amount of oxygen supply, which is a major factor related to the amount of heat production, is expressed as the product of hemoglobin concentration, hemoglobin oxygen saturation, and blood flow volume.

The hemoglobin concentration can be measured from the absorbance at the wavelength (equal-absorbance wavelength) at which the molar absorbance coefficient of the oxyhemoglobin is equal to that of the reduced (deoxy-) hemoglobin. The hemoglobin oxygen saturation can be measured by measuring the absorbance at the equal-absorbance wavelength and the absorbance at at least one different wavelength at which the ratio between the molar absorbance coefficient of the oxyhemoglobin and that of the reduced (deoxy-) hemoglobin is known, and then solving simultaneous equations. Namely, the hemoglobin concentration and hemoglobin oxygen saturation can be obtained by conducting the measurement of absorbance at at least two wavelengths.

Since the hemoglobin concentration measured through the skin is the blood hemoglobin concentration inside the skin tissue on which light has been incident and through which the light has been transmitted, it is proportional to the amount of blood inside the tissue. It is also proportional to the blood hemoglobin concentration inside a collected blood sample that is measured accurately by a method such as spectrophotometry. Therefore, the blood hemoglobin concentration inside the tissue is proportional to the product of the blood amount inside the tissue and the blood hemoglobin concentration inside a blood sample.

The blood flow volume is considered next. In a steady state, when the velocity of blood in capillary blood vessels is considered constant, the blood flow volume is proportional to the amount of blood inside tissue.

From the above discussion, the blood flow volume is proportional to (blood hemoglobin concentration inside tissue/blood hemoglobin concentration inside a blood sample).

Thus, it will be seen that the measured quantities necessary for the determination of blood glucose concentration by the above-described model are the room temperature (ambient temperature), the temperature due to radiation from the body surface, the absorbance at at least two wavelengths, and the blood hemoglobin concentration inside a blood sample that is measured separately.

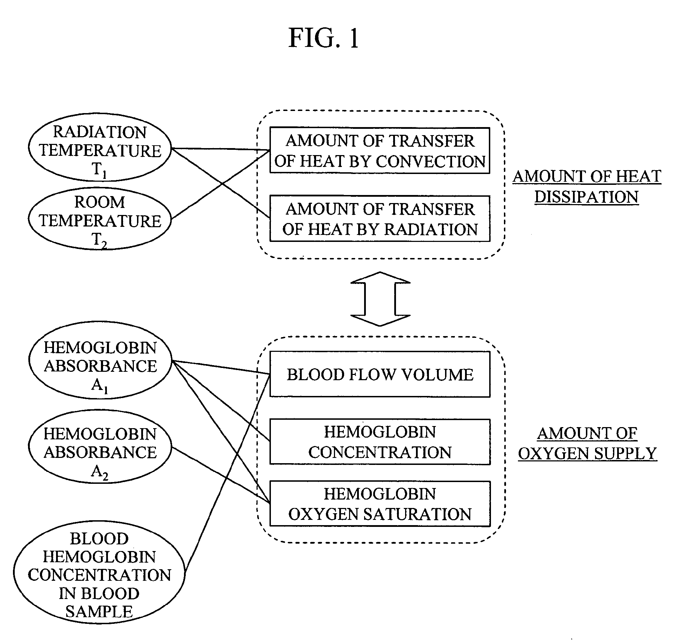

Fig. 1 shows the relationships between the measured values provided by various sensors and the parameters derived therefrom. Radiation temperature $T_1$ on the body surface and room temperature $T_2$ are measured. Absorbance $A_1$ and $A_2$ are measured at at least two wavelengths related to the absorption of hemoglobin. The temperature $T_1$ provides a parameter related to the amount of heat transferred by radiation. The temperatures $T_1$ and $T_2$ provide parameters related to the amount of heat transferred by convection. The absorbance $A_1$ provides a parameter related to the hemoglobin concentration, and the absorbance $A_1$ and $A_2$ provide parameters related to the hemoglobin oxygen saturation. The absorbance $A_1$ and the blood hemoglobin concentration inside a blood sample that is measured in advance provide parameters related to the blood flow volume.

Hereafter, an example of an apparatus for non-invasively measuring blood sugar levels according to the principle of the invention will be described.

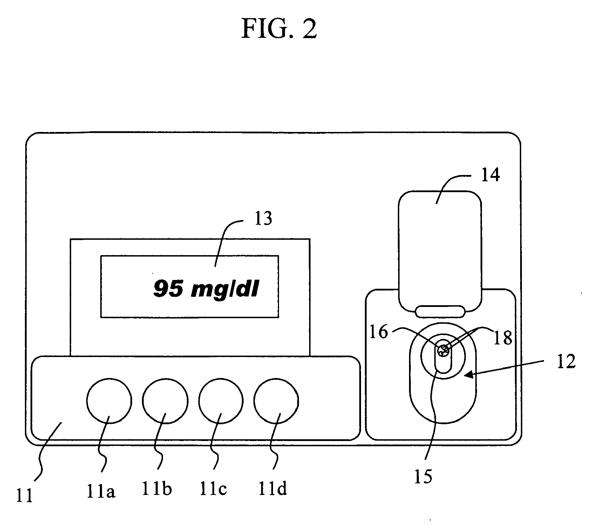

Fig. 2 shows a top plan view of a non-invasive blood sugar level measuring apparatus according to the invention. While in this example the skin on the ball of the fingertip is used as the body surface, other parts of the body surface may be used.

On the top surface of the apparatus are provided an operating portion 11, a measuring portion 12 where the finger to be measured is to be placed, and an LCD 13 for displaying measurement results, the state of the apparatus, measured values, for example. The operating portion 11 includes four push buttons 11 a to 11d for operating the apparatus. The measuring portion 12 has a cover 14 which, when opened (as shown), reveals a finger rest portion 15 with an oval periphery. The finger rest portion 15 accommodates an opening end 16 of a radiation-temperature sensor portion, and an optical sensor portion 18.

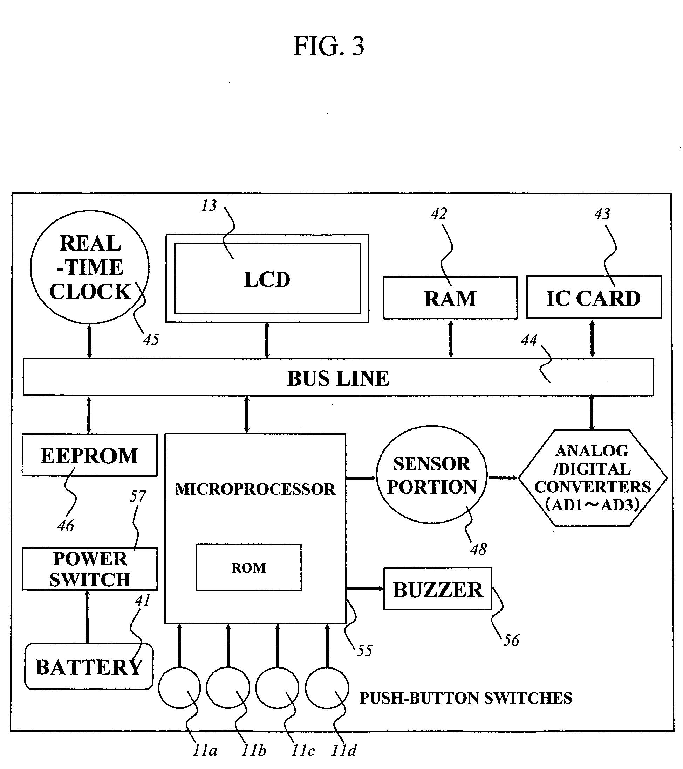

Fig. 3 shows a functional block diagram of the apparatus, which is powered by a battery 41, whose voltage supply is switched on or off by a power switch 57. A microprocessor 55 includes a ROM for the storage of software. A sensor portion 48 consists of a temperature sensor and an optical sensor. Two light-emitting diodes as the light source for the optical sensors are caused to emit light in a time-shared manner by the microprocessor 55. Signals measured by the sensor portion 48 are supplied to analog/digital converters AD1 to AD3 disposed in such a way as to correspond to individual sensors, where the signals are converted into digital signals. The digital signals are fed to the microprocessor 55 and stored in a RAM 42. Peripheral circuits to the microprocessor 55 include the analog/digital converters AD1 to AD3, LCD 13, RAM 42, IC card 43, real-time clock 45, and EEPROM 46. These peripheral circuits are accessed by the microprocessor 55 via a bus line 44. Push buttons 11a to 11d are individually connected to the microprocessor 55. A buzzer 56 is connected to the microprocessor 55 such that it can be turned on or off by the control of the microprocessor 55.

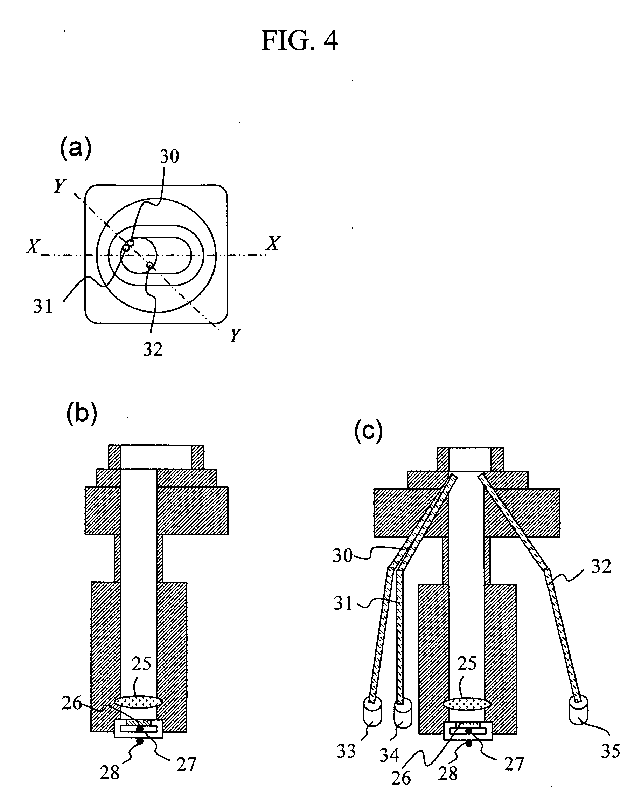

Fig. 4 shows the structure of the measuring portion 12. In Fig. 4, (a) is a top plan view, (b) is a cross section taken along line X-X of (a), and (c) is a cross section taken along line Y-Y of (a).

Hereafter, the temperature sensors and the optical sensor portion 18 in the measuring portion 12 are described. First, the temperature sensors are described. An infrared lens 25 is disposed inside the apparatus at such a position that the examined portion (ball of the finger) placed on the finger rest portion 15 can be seen through the lens. Below the infrared lens 25 is disposed a thermopile 27 via an infrared radiation-transmitting window 26. A platinum temperature-measuring resistor 28 is disposed in close proximity to the thermopile 27.

Thus, the temperature sensor portion of the measuring portion includes two temperature sensors, which measure the following two kinds of temperatures.

(1) Temperature of radiation from the finger (thermopile 27): $T_1$
(2) Room temperature (platinum temperature-measuring resistor 28): $T_2$

The optical sensor portion 18 is described hereafter. The optical sensor portion 18 measures the hemoglobin concentration and the hemoglobin oxygen saturation necessary for the determination of the oxygen supply volume, and the blood flow volume. In order to measure the hemoglobin concentration, hemoglobin oxygen saturation, and the blood flow volume, it is necessary to measure the absorbance at at least two wavelengths. Fig. 4(c) shows an example for carrying out a two-wavelength measurement using two light sources 33 and 34 and one detector 35.

The ends of three optical fibers 30, 31 and 32 are located in the optical sensor portion 18. The optical fibers 30 and 31 are for optical irradiation, while the optical fiber 32 is for receiving light. As shown in Fig. 4(c), the optical fibers 30 and 31 are provided at the ends thereof with the light-emitting diodes 33 and 34, respectively, of two wavelengths. The light-receiving optical fiber 32 is provided at the end thereof with the photodiode 35. The light-emitting diode 33 emits light of the wavelength of 810 nm, while the light-emitting diode 34 emits light of the wavelength of 950 nm. The wavelength 810 nm is the equal absorbance wavelength at which the molar absorbance coefficient of the oxyhemoglobin is equal to that of the reduced (deoxy-) hemoglobin. The wavelength 950 nm is the wavelength at which the difference between the molar absorbance coefficient of the oxyhemoglobin and that of the reduced hemoglobin is large.

The two light-emitting diodes 33 and 34 emit light in a time-sharing manner. The finger of an examined subject is irradiated with the light emitted by the light-emitting diodes 33 and 34 via the light-irradiating fibers 30 and 31. The light shone on the finger is reflected by the skin of the finger and is then incident on the light-receiving optical fiber 32, via which the light is detected by the photodiode 35. When the light with which the finger is irradiated is reflected by the skin of the finger, part of the light penetrates into the tissue through the skin and is absorbed by the hemoglobin in the blood that flows in capillary blood vessels. The measurement data provided by the photodiode 35 is reflectance R. Absorbance can be approximately calculated from log(1/R). Irradiation is conducted with light of wavelengths 810 nm and 950 nm, R is measured for each, and then log(1/R) is obtained, thereby measuring absorbance $A_1$ for wavelength 810 nm and absorbance $A_2$ for wavelength 950 nm.

When the reduced hemoglobin concentration is [Hb] and the oxyhemoglobin concentration is [HbO$_2$], absorbance $A_1$ and absorbance $A_2$ are expressed by the following equations:

$$A_1 = a \times ([Hb] \times A_{Hb}(810nm) + [HbO_2] \times A_{HbO_2}(810nm))$$

$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$

$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

where $A_{Hb}$(810 nm) and $A_{Hb}$(950 nm), and $A_{Hb02}$(810 nm) and $A_{Hb02}$(950 nm) are the molar absorbance coefficients of the reduced hemoglobin and the oxyhemoglobin, respectively, and are known at the respective wavelengths. The term $a$ is a proportionality coefficient. From the above equations, the blood hemoglobin concentration ([Hb]+[HbO$_2$])$_T$ inside tissue and the blood hemoglobin oxygen saturation ([HbO$_2$]/([Hb]+[HbO$_2$]))$_T$ are determined as follows:

$$([Hb] + [HbO_2])_T = \frac{A_1}{a \times A_{HbO_2}(810nm)}$$

$$\left( \frac{[HbO_2]}{[Hb] + [HbO_2]} \right)_T = \frac{A_2 \times A_{HbO_2}(810nm) - A_1 \times A_{Hb}(950nm))}{A_1 \times (A_{HbO_2}(950nm) - A_{Hb}(950nm))}$$

While the above example involved the measurement of the blood hemoglobin concentration and hemoglobin oxygen saturation inside tissue based on the measurement of absorbance at two wavelengths, it is also possible to reduce the influence of interfering components and increase measurement accuracy by measuring absorbance at three or more wavelengths.

Because the blood flow volume BF inside tissue is proportional to the ratio between the blood hemoglobin concentration ([Hb]+[HbO$_2$])$_T$ inside tissue and the blood hemoglobin concentration ([Hb]+[HbO$_2$])$_B$ inside a blood sample, it is expressed by the following equation:

$$BF \propto \frac{([Hb] + [HbO_2])_T}{([Hb] + [HbO_2])_B} = \frac{A_1}{a \times A_{HbO_2}(810nm) \times ([Hb] + [HbO_2])_B}$$

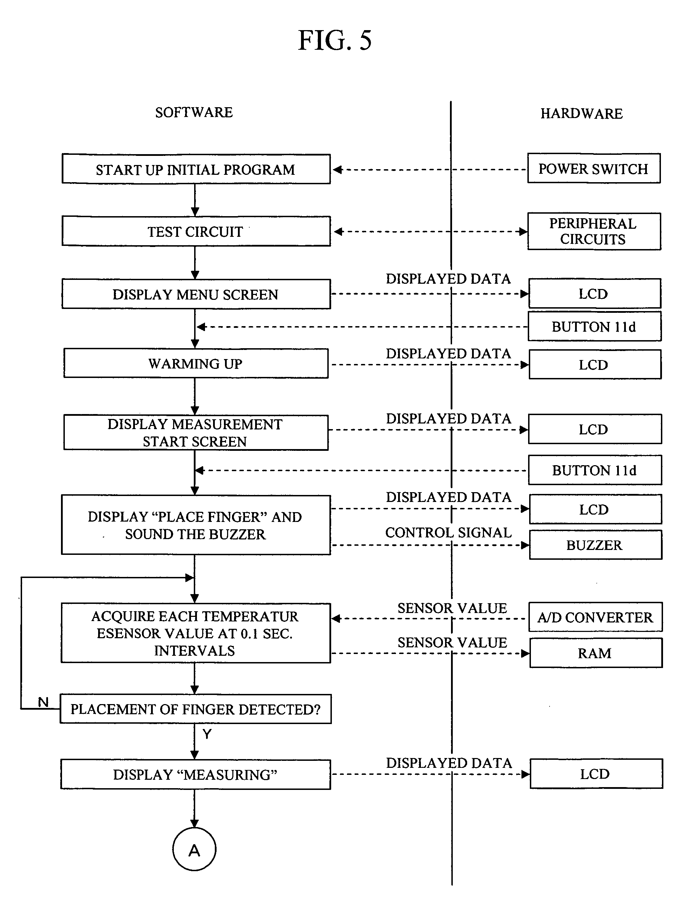

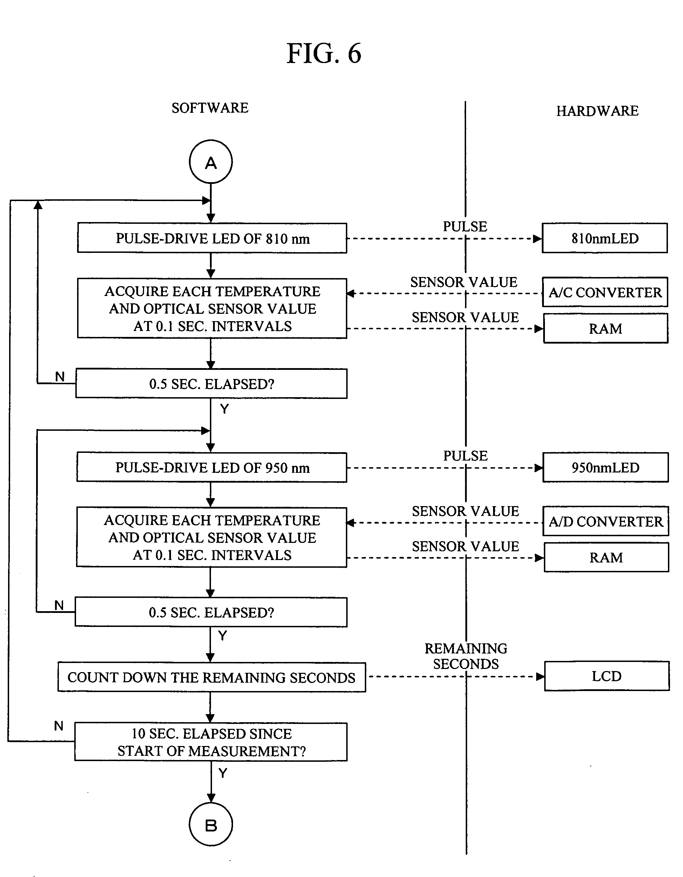

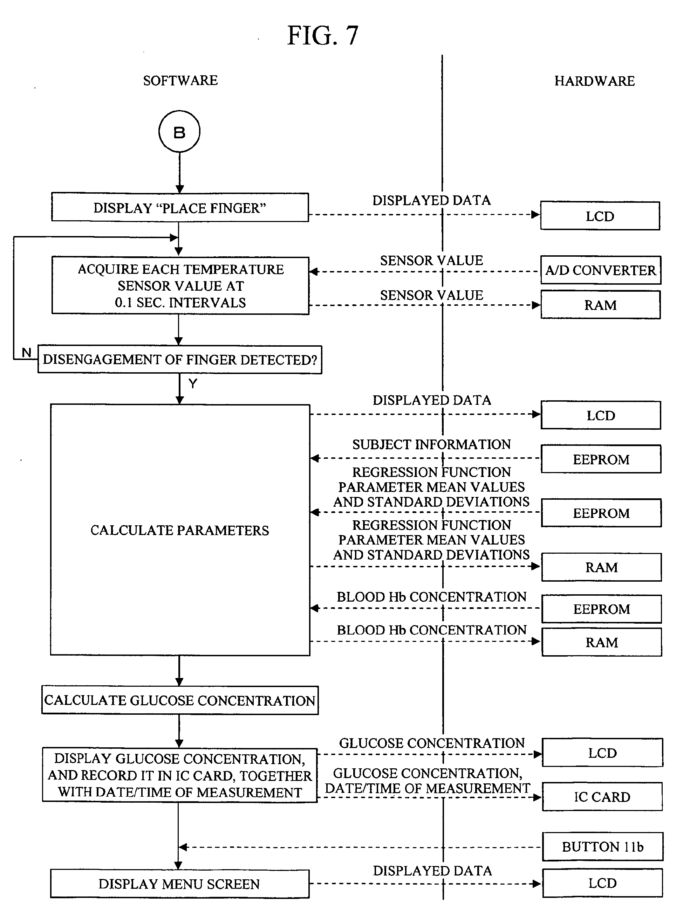

Figs. 5, 6, and 7 show the flow of software and hardware operations during the measurement of glucose concentrations. As the power switch 57 is pressed and the microprocessor 55 is supplied with power, the initial program is activated and then the function of each of the circuits peripheral to the microprocessor 55 is tested. After the circuit test is finished, a menu screen is displayed on the LCD 13. If the subject presses the button 11d at this point, the measurement mode is initiated and a warm-up starts. After the warm-up is over, a measurement start screen is displayed. If the subject presses the button 11d, the LCD 13 displays "PLACE FINGER," and the buzzer sounds. In a finger-placement waiting state, data from the temperature sensor is obtained via the A/D converter at 0.1 second intervals in order to determine whether the finger is placed based on the change in $T_1$. When the finger is placed on the finger rest, the LCD 13 displays "MEASURING," and the measurement operation is started.

In the measurement operation, after the 810 nm LED is pulse-driven, values from the temperature sensor and the optical sensor 18 are obtained via the A/D converters at 0.1 second intervals and then stored in RAM 42. This is carried out in 0.5 second. Then, the 950 nm LED is pulse-driven, and values from the temperature sensor and the optical sensor 18 are obtained via the A/D converters at 0.1 second intervals and stored in RAM 42. After performing this in 0.5 second, the remaining seconds being displayed on the LCD 13 are counted down. The above measurement operation is conducted until 10 seconds elapses from the start of measurement.

When the measurement operation is over, the LCD 13 displays "PUT FINGER AWAY," and the buzzer sounds. In a finger-disengagement waiting state, data is obtained from each temperature sensor at 0.1 second intervals in order to determine whether the finger has been disengaged based on the change in $T_1$.

As the finger is detached from the finger rest portion, parameter calculations are carried out by software based on the temperature sensor values and optical sensor values that have been acquired during the time when the finger was placed on the finger rest. The software reads from EEPROM 46 subject information that has been stored in advance and which indicates whether the subject is an "able-bodied person" or a "diabetic patient." If the subject is an "able-bodied person," the software proceeds to read from EEPROM 46 the regression function, parameter mean values, and standard deviations for able-bodied persons, and stores them in RAM 42. On the other hand, if the subject is a "diabetic patient," the software reads from EEPROM 46 the regression function, parameter mean values, and standard deviations for diabetic patients, and stores them in RAM 42. The software further reads from EEPROM 46 the blood hemoglobin concentration inside a blood sample that has been measured and stored in advance, and stores it in RAM 42. The blood hemoglobin concentration inside blood sample stored in RAM 42 is used for the calculation of a parameter relating to the blood flow volume. Following the parameter calculation, a glucose concentration calculation is conducted. A calculated glucose concentration is displayed on the LCD 13, and is also recorded in the IC card together with the date and time of measurement. When the button 11d is pressed, the flow returns to the measurement start screen.

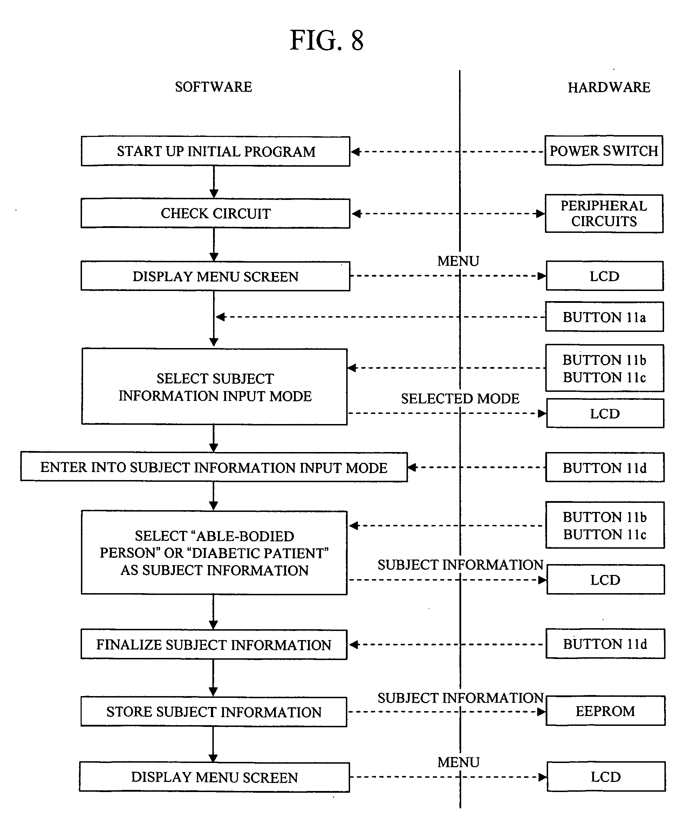

Fig. 8 shows the flow of software and hardware operations during the input of subject information. The operations up to and including the display of the menu screen are the same as those for the measurement of blood sugar levels. When the button 11a is pressed on the menu screen, a setting mode is initiated. In the setting mode, it is possible to set the date/time, refer to the past blood sugar level history data, set the subject information, and set the blood hemoglobin concentration inside a blood sample. As a subject information setting mode is selected by the buttons 11b and 11c and then the button 11d is pressed, the subject information setting mode is initiated. Set values are finalized by selecting either the "able-bodied person" or the "diabetic patient" using the buttons 11b and 11c and then pressing the button 11d. The thus finalized subject information is stored in EEPROM 46, and then the flow returns to the menu screen.

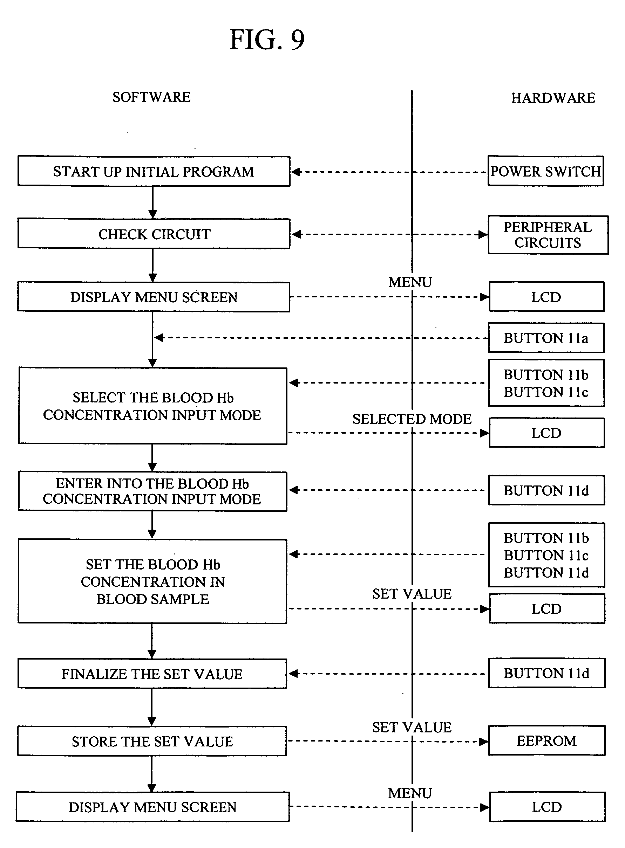

Fig. 9 shows the flow of software and hardware operations during the input of the blood hemoglobin concentration in a blood sample. The operation up to the transition to the setting mode is the same as that for the input of subject information. After selecting a blood hemoglobin concentration setting mode using the buttons 11b and 11c, the blood hemoglobin concentration setting mode can be initiated by pressing the button 11d. Values are then set using the buttons 11b and 11c, which values are then finalized by pressing the button 11d. The thus finalized values are stored in EEPROM 46, and the flow returns to the menu screen. The measurement of the blood hemoglobin concentration based on the collection of a blood sample and using a spectrophotometer, and the updating of the blood hemoglobin concentration values stored in EEPROM 46, may be performed at the frequency of once or twice per month.

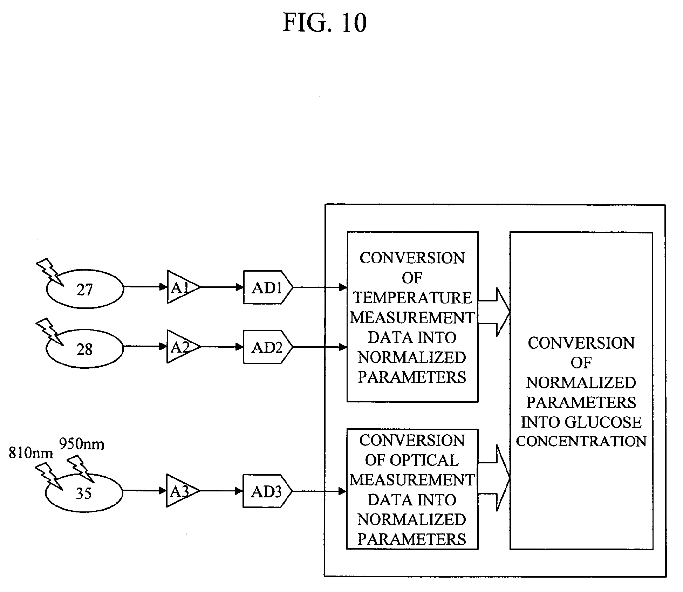

Fig. 10 is a conceptual chart showing the flow of data processing in the apparatus. The apparatus according to the present example includes three sensors, namely a thermopile 27, a platinum temperature-measuring resistor 28, and a photodiode 35. The photodiode 35 measures absorbance at wavelengths 810 nm and 950 nm. Thus, the apparatus is supplied with four kinds of measurement values.

The three kinds of analog signals are supplied via individual amplifiers A1 to A3 to analog/digital converters AD1 to AD3, where they are converted into digital signals. Based on the digitally converted values, parameters $x_i$ (i=1, 2, 3, 4, 5) are calculated. The following are specific descriptions of $x_i$ (where $a_1$ to $a_5$ are proportionality coefficients):

Parameter proportional to heat radiation

$$x_1 = a_1 \times (T_1)^4$$

Parameter proportional to heat convection

$$x_2 = a_2 \times (T_2 - T_1)$$

Parameter proportional to the hemoglobin concentration inside tissue

$$x_3 = a_3 \times \left( \frac{A_1}{A_{HbO_2}(810nm)} \right)$$

Parameter proportional to the hemoglobin saturation inside tissue

$$x_4 = a_4 \times \left( \frac{A_2 \times A_{HbO_2}(810nm) - A_1 \times A_{Hb}(950nm))}{A_1 \times (A_{HbO_2}(950nm) - A_{Hb}(950nm))} \right)$$

Parameter proportional to the blood flow volume inside tissue

$$x_5 = a_5 \times \left( \frac{A_1}{A_{HbO_2}(810nm) \times ([Hb] + [HbO_2])_B} \right)$$

Then, normalized parameters are calculated from mean values and standard deviations of parameter $x_i$ obtained from actual data pertaining to large numbers of able-bodied people and diabetic patients. A normalized parameter $X_i$ (where i=1, 2, 3, 4, 5) is calculated from each parameter $x_i$ according to the following equation:

$$X_i = \frac{x_i - \bar{x}_i}{SD(x_i)}$$

where

$x_i$: parameter

$\bar{x}_i$ : : mean value of the parameter

$SD(x_i)$: standard deviation of the parameter

Using the above five normalized parameters, calculations are conducted for conversion into a glucose concentration to be eventually displayed. A program necessary for the processing calculations is stored in a ROM in the microprocessor built inside the apparatus. A memory area required for the processing calculations is secured in RAM 42 similarly built inside the apparatus. The result of the calculations is displayed on the LCD.

EEPROM 46 stores, as a constituent element of the program necessary for the processing calculations, a regression function for determining glucose concentration C in particular. The regression function is determined in advance for the diabetic patients and the able-bodies persons individually by the least-squares method using the glucose concentrations measured from a large number of diabetic patients and able-bodied persons by the enzyme electrode method, which is an invasive method, and using the normalized parameters simultaneously obtained from the large number of diabetic patients and able-bodied persons.

A method of determining the regression function is described below, with reference to a regression function for diabetic patients as an example. The glucose concentration $C_D$ is expressed by the below-indicated equation (1), where $a_{Di}$ (i=0, 1, 2, 3, 4, 5) is determined from measurement data on a plurality of diabetic patients in advance according to the following procedure:

(1) A multiple regression equation is created that indicates the relationship between the normalized parameters and the glucose concentration $C_D$.
(2) Normalized equations (simultaneous equations) relating to the normalized parameters are obtained from equations obtained by the least-squares method.
(3) Values of coefficient $a_{Di}$ (i=0, 1, 2, 3, 4, 5) are determined from the normalized equations and are then substituted into the multiple regression equation.

Initially, the regression equation (1) indicating the relationship between the glucose concentration $C_D$ for the diabetic patients and the normalized parameters $X_{D1}$, $X_{D2}$, $X_{D3}$, $X_{D4}$, and $X_{D5}$ is formulated.

$$C_D = f(X_{D1}, X_{D2}, X_{D3}, X_{D4}, X_{D5})$$
$$= a_{D0} + a_{D1}X_{D1} + a_{D2}X_{D2} + a_{D3}X_{D3} + a_{D4}X_{D4} + a_{D5}X_{D5} \quad ......(1)$$

Then, the least-squares method is employed to obtain a multiple regression equation that would minimize the error with respect to a measured value $C_{Di}$ of glucose concentration according to the enzyme electrode method. When the sum of the squares of the residual is $R_D$, $R_D$ is expressed by the following equation (2):

$$R_D = \sum_{i=1}^{n} d_{Di}^{2}$$
$$= \sum_{i=1}^{n} (C_{Di} - f(X_{D1i}, X_{D2i}, X_{D3i}, X_{D4i}, X_{D5i}))^2$$
$$= \sum_{i=1}^{n} \{C_{Di} - (a_{D0} + a_{D1}X_{D1i} + a_{D2}X_{D2i} + a_{D3}X_{D3i} + a_{D4}X_{D4i} + a_{D5}X_{D5i})\}^2 \quad ......(2)$$

The sum $R_D$ of the squares of the residual becomes minimum when partial differentiation of equation (2) with respect to $a_{D0}$, $a_{D1}$,..., $a_{D5}$ gives zero. Thus, we have the following equations:

$$\frac{\partial R_D}{\partial a_{D0}} = -2\sum_{i=1}^{n} \{C_{Di} - (a_{D0} + a_{D1}X_{D1i} + a_{D2}X_{D2i} + a_{D3}X_{D3i} + a_{D4}X_{D4i} + a_{D5}X_{D5i})\} = 0$$

$$\frac{\partial R_D}{\partial a_{D1}} = -2\sum_{i=1}^{n} X_{D1i}\{C_{Di} - (a_{D0} + a_{D1}X_{D1i} + a_{D2}X_{D2i} + a_{D3}X_{D3i} + a_{D4}X_{D4i} + a_{D5}X_{D5i})\} = 0$$

$$\frac{\partial R_D}{\partial a_{D2}} = -2\sum_{i=1}^{n} X_{D2i}\{C_{Di} - (a_{D0} + a_{D1}X_{D1i} + a_{D2}X_{D2i} + a_{D3}X_{D3i} + a_{D4}X_{D4i} + a_{D5}X_{D5i})\} = 0$$

$$\frac{\partial R_D}{\partial a_{D3}} = -2\sum_{i=1}^{n} X_{D3i}\{C_{Di} - (a_{D0} + a_{D1}X_{D1i} + a_{D2}X_{D2i} + a_{D3}X_{D3i} + a_{D4}X_{D4i} + a_{D5}X_{D5i})\} = 0$$

$$\frac{\partial R_D}{\partial a_{D4}} = -2\sum_{i=1}^{n} X_{D4i}\{C_{Di} - (a_{D0} + a_{D1}X_{D1i} + a_{D2}X_{D2i} + a_{D3}X_{D3i} + a_{D4}X_{D4i} + a_{D5}X_{D5i})\} = 0$$

$$\frac{\partial R_D}{\partial a_{D5}} = -2\sum_{i=1}^{n} X_{D5i}\{C_{Di} - (a_{D0} + a_{D1}X_{D1i} + a_{D2}X_{D2i} + a_{D3}X_{D3i} + a_{D4}X_{D4i} + a_{D5}X_{D5i})\} = 0 \quad ......(3)$$

When the mean values of $C_{Dmean}$ and $X_{D1}$ to $X_{D5}$ are $C_{Dmean}$ and $X_{D1mean}$ to $X_{D5mean}$, respectively, since $X_{Dimean}=0$ (i=1 to 5), equation (1) yields:

$$a_{D0} = C_{Dmean} - a_{D1}X_{D1mean} - a_{D2}X_{D2mean} - a_{D3}X_{D3mean} - a_{D4}X_{D4mean} - a_{D5}X_{D5mean}$$
$$= C_{Dmean} \quad ......(4)$$

The variation and covariation between the normalized parameters are expressed by equation (5). Covariation between the normalized parameter $X_{Di}$ (i=1 to 5) and $C_D$ is expressed by equation (6).

$$S_{Dij} = \sum_{k=1}^{n} (X_{Dik} - X_{Dimean})(X_{Djk} - X_{Djmean}) = \sum_{k=1}^{n} X_{Dik} X_{Djk} \quad (i,j=1,2,..5) \quad ......(5)$$

$$S_{DiC} = \sum_{k=1}^{n} (X_{Dik} - X_{Dimean})(C_{Dk} - C_{Dmean}) = \sum_{k=1}^{n} X_{Dik} (C_{Dk} - C_{Dmean}) \quad (i=1,2,..5) \quad ......(6)$$

Substituting equations (4), (5), and (6) into equation (3) and rearranging yields a set of simultaneous equations (normalized equations) (7). Solving the set of equations (7) yields $a_{D1}$ to $a_{D5}$.

$$a_{D1}S_{D11} + a_{D2}S_{D12} + a_{D3}S_{D13} + a_{D4}S_{D14} + a_{D5}S_{D15} = S_{D1C}$$
$$a_{D1}S_{D21} + a_{D2}S_{D22} + a_{D3}S_{D23} + a_{D4}S_{D24} + a_{D5}S_{D25} = S_{D2C}$$
$$a_{D1}S_{D31} + a_{D2}S_{D32} + a_{D3}S_{D33} + a_{D4}S_{D34} + a_{D5}S_{D35} = S_{D3C}$$
$$a_{D1}S_{D41} + a_{D2}S_{D42} + a_{D3}S_{D43} + a_{D4}S_{D44} + a_{D5}S_{D45} = S_{D4C}$$
$$a_{D1}S_{D51} + a_{D2}S_{D52} + a_{D3}S_{D53} + a_{D4}S_{D54} + a_{D5}S_{D55} = S_{D5C} \quad ......(7)$$

Constant term $a_{D0}$ is determined by means of equation (4). The thus obtained $a_{Di}$ (i=0, 1, 2, 3, 4, 5) is stored in EEPROM 46 at the time of manufacture of the apparatus. In actual measurement using the apparatus, the normalized parameters $X_{D1}$ to $X_{D5}$ obtained from the measured values are substituted into regression equation (1) to calculate the glucose concentration $C_D$.

Similarly, the coefficient $a_{Ni}$ (i=0, 1, 2, 3, 4, 5) for the able-bodied persons is determined in advance from measurement data on a number of able-bodied persons and is then stored in EEPROM 46 as a regression function (8) for the able-bodied persons.

$$C_N = f(X_{N1}, X_{N2}, X_{N3}, X_{N4}, X_{N5})$$
$$= a_{N0} + a_{N1}X_{N1} + a_{N2}X_{N2} + a_{N3}X_{N3} + a_{N4}X_{N4} + a_{N5}X_{N5} \quad ......(8)$$

Hereafter, a concrete example of the process of calculating the glucose concentration is described. The coefficients for regression equation (1) are determined from a number of pieces of data measured in advance from diabetic patients, and EEPROM 46 stores the following formula for the calculation of the glucose concentration. In EEPROM 46, there are further stored the mean values and standard deviations of the parameters $x_1$ to $x_5$.

$$C_D = 215.5 - 22.8 \times X_{D1} + 26.5 \times X_{D2} - 14.1 \times X_{D3} - 12.6 \times X_{D4} - 24.3 \times X_{D5} \quad ......(9)$$

Similarly, a glucose-concentration calculation formula (10) for the able-bodied persons, and the mean values and standard deviations of the parameters $x_1$ to $x_5$ are stored in EEPROM 46.

$$C_N = 96.2 + 12.6 \times X_{N1} - 14.4 \times X_{N2} - 1.9 \times X_{N3} - 1.3 \times X_{N4} + 13.8 \times X_{N5} \quad ......(10)$$

$X_{D1}$ to $X_{D5}$ are obtained by normalizing parameters $x_1$ to $x_5$ using the mean values and standard deviations of the diabetic patients. $X_{N1}$ to $X_{N5}$ are obtained by normalizing parameters $x_1$ to $x_5$ using the mean values and standard deviations of the able-bodied persons. Assuming the distribution of a parameter is normal, 95% of a normalized parameter takes on values between -2 and +2.

The following describes the results of measurement by the conventional enzymatic electrode method, in which a blood sample is reacted with a reagent and the amount of resultant electrons is measured to determine glucose concentration, and the results of measurement by an embodiment of the invention. When the measured value of glucose concentration for a diabetic patient was 257 mg/dl according to the enzymatic electrode method, substituting the normalized parameters $X_1$=-0.35, $X_2$=+0.28, $X_3$=-0.09, $X_4$=-0.14, and $X_5$=-0.29, which were obtained by measurement at the same time according to the invention, into above equation (9) yields $C_D$=241 mg/dl.

In another example of the measured value for an able-bodied person, when the glucose concentration was 88 mg/dl according to the enzymatic electrode method, substituting the normalized parameters $X_{N1}$=-0.19, $X_{N2}$=+0.14, $X_{N3}$=+0.08, $X_{N4}$=+0.11, and $X_{N5}$=-0.13, which were obtained by measurement at the same time according to the invention, into above equation (10) yields $C_N$=90 mg/dl. The results thus indicated that the method according to the invention can provide highly accurate glucose concentration values.

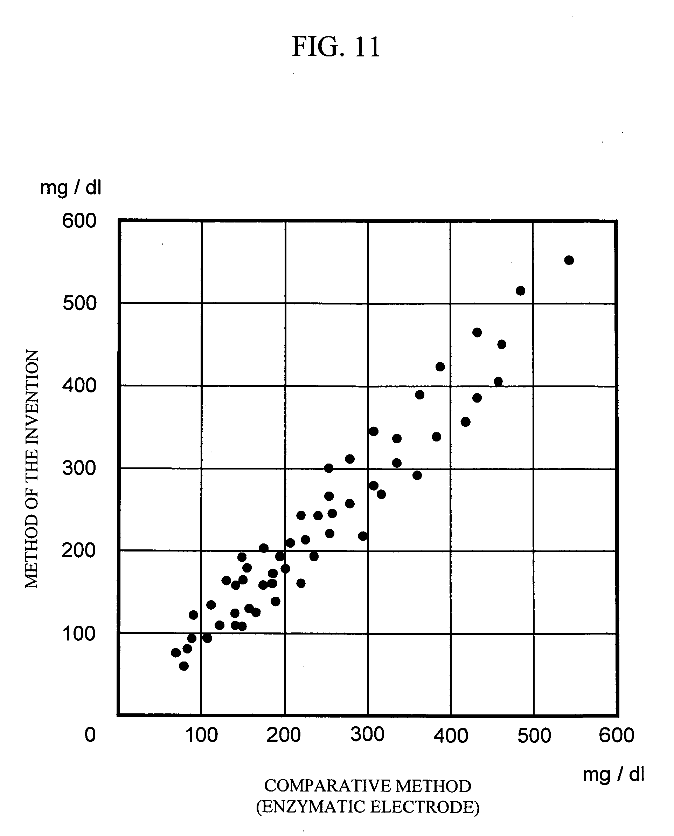

Fig. 11 shows a graph plotting glucose concentrations for a plurality of diabetic patients and able-bodied persons, with the vertical axis showing the calculated values of glucose concentration according to the invention, and the horizontal axis showing the measured values of glucose concentration according to the enzymatic electrode method. It is seen that a good correlation is obtained by measuring the oxygen supply volume and the blood flow volume according to the method of the invention (correlation coefficient=0.9194).

## Claims

1. A blood sugar level measuring apparatus comprising:

   a heat amount measuring portion for measuring a temperature deriving from a body surface in order to obtain information used for the calculation of the amount of heat transfer due to convection and the amount of heat transfer due to radiation, which are related to the dissipation of heat from said body surface;
   an optical measuring portion for irradiating said body surface with light in order to obtain measurement values related to the blood hemoglobin concentration and the hemoglobin oxygen saturation inside tissue;
   a memory portion for storing a measurement value relating to the blood hemoglobin concentration measured using a blood sample that is separately collected, and for storing relationships between parameters corresponding to said temperature, said blood hemoglobin concentration and said hemoglobin oxygen saturation inside tissue, the volume of blood flow inside tissue, and blood sugar levels;
   a calculation portion for converting a plurality of measurement values inputted from said heat amount measuring portion and said optical measuring portion, into said parameters individually, and for calculating a blood sugar level by applying said parameters to said relationships stored in said memory portion; and
   a display portion for displaying the result of calculation in said calculation portion, wherein
   the parameter corresponding to said blood flow volume inside tissue is generated on the basis of a ratio between the blood hemoglobin concentration inside tissue and the blood hemoglobin concentration measured using said blood sample.

2. The blood sugar level measuring apparatus according to claim 1, wherein said optical measuring portion comprises:

   a light source for producing light of at least two different wavelengths;
   an optical system for irradiating said body surface with light emitted by said light source; and
   a photodetector for detecting reflected light from said body surface.

3. The blood sugar level measuring apparatus according to claim 2, wherein said optical measuring portion comprises:

   a light source for emitting light of the wavelength of substantially 810 nm; and
   a light source for emitting light of the wavelength of substantially 950 nm.

4. The blood sugar level measuring apparatus according to claim 1, wherein said heat amount measuring portion comprises:

   an ambient temperature detector for measuring ambient temperature; and
   a radiation temperature detector for measuring radiation heat from said body surface.

5. A blood sugar level measuring apparatus comprising:

an ambient temperature measuring portion for measuring ambient temperature;

a radiation heat detector for measuring radiation heat from a body surface;

a light source for irradiating said body surface with at least light of a first wavelength and light of a second wavelength;

a photodetector for detecting reflected light that is produced as said light from said light source is reflected by said body surface;

a memory portion for storing a measurement value relating to the blood hemoglobin concentration that is measured using a blood sample that is separately collected;

a calculating portion including a converting portion for converting said stored measurement value and the individual outputs of said ambient temperature measuring portion, said radiation heat detector, and said photodetector into a plurality of parameters, and a processing portion in which relationships between said parameters and blood sugar levels are stored, said processing portion calculating a blood sugar level by applying said parameters to said relationships; and

a display portion for displaying a result outputted from said calculating portion.

6. The blood sugar level measuring apparatus according to claim 5, wherein said stored measurement value is associated with the output of said photodetector when converted into said parameter.

7. The blood sugar level measuring apparatus according to claim 5, wherein said stored measurement value is converted into said parameter based on its ratio to the output of said photodetector.

8. The blood sugar level measuring apparatus according to claim 5, further comprising an infrared lens between an open end through which said light source emits said light of said first wavelength and said second wavelength and said radiation heat detector.

9. The blood sugar level measuring apparatus according to claim 7, further comprising an infrared light transmitting window between said infrared lens and said radiation heat detector.

10. The blood sugar level measuring apparatus according to claim 5, wherein said ambient temperature measuring portion comprises a temperature-measuring resistor disposed near said radiation heat detector.

11. The blood sugar level measuring apparatus according to claim 5, wherein said first wavelength and said second wavelength are substantially 810 nm and substantially 950 nm, respectively.

# FIG. 1

RADIATION TEMPERATURE $T_1$

ROOM TEMPERATURE $T_2$

AMOUNT OF TRANSFER OF HEAT BY CONVECTION

AMOUNT OF TRANSFER OF HEAT BY RADIATION

AMOUNT OF HEAT DISSIPATION

HEMOGLOBIN ABSORBANCE $A_1$

HEMOGLOBIN ABSORBANCE $A_2$

BLOOD HEMOGLOBIN CONCENTRATION IN BLOOD SAMPLE

BLOOD FLOW VOLUME

HEMOGLOBIN CONCENTRATION

HEMOGLOBIN OXYGEN SATURATION

AMOUNT OF OXYGEN SUPPLY

13

FIG. 2

95 mg/dl

# FIG. 3

# FIG. 4

(a)

(b)

(c)

# FIG. 5

SOFTWARE | HARDWARE

```
┌──────────────────────────┐          ┌──────────────────┐
│ START UP INITIAL PROGRAM │◄─ ─ ─ ─ ─│   POWER SWITCH   │
└──────────────────────────┘          └──────────────────┘
            │
            ▼
┌──────────────────────────┐          ┌──────────────────┐
│       TEST CIRCUIT       │◄─ ─ ─ ─ ─│    PERIPHERAL    │
└──────────────────────────┘          │     CIRCUITS     │
            │                          └──────────────────┘
            ▼              DISPLAYED DATA
┌──────────────────────────┐          ┌──────────────────┐
│   DISPLAY MENU SCREEN    │─ ─ ─ ─ ─►│       LCD        │
└──────────────────────────┘          └──────────────────┘
            │◄ ─ ─ ─ ─ ─ ─ ─ ─ ─ ─ ─ ─│    BUTTON 11d    │
            ▼              DISPLAYED DATA└──────────────────┘
┌──────────────────────────┐          ┌──────────────────┐
│        WARMING UP        │─ ─ ─ ─ ─►│       LCD        │
└──────────────────────────┘          └──────────────────┘
            │              DISPLAYED DATA
            ▼
┌──────────────────────────┐          ┌──────────────────┐
│   DISPLAY MEASUREMENT    │─ ─ ─ ─ ─►│       LCD        │
│      START SCREEN        │          └──────────────────┘
└──────────────────────────┘          ┌──────────────────┐
            │◄ ─ ─ ─ ─ ─ ─ ─ ─ ─ ─ ─ ─│    BUTTON 11d    │
            ▼              DISPLAYED DATA└──────────────────┘
┌──────────────────────────┐          ┌──────────────────┐
│ DISPLAY "PLACE FINGER" AND│─ ─ ─ ─ ─►│       LCD        │
│    SOUND THE BUZZER      │          └──────────────────┘
└──────────────────────────┘  CONTROL SIGNAL┌─────────────┐
            │          ─ ─ ─ ─ ─ ─ ─ ─ ─►│    BUZZER    │
            ▼                          └──────────────────┘
┌──────────────────────────┐  SENSOR VALUE┌─────────────────┐
│  ACQUIRE EACH TEMPERATUR │◄─ ─ ─ ─ ─│  A/D CONVERTER   │
│  ESENSOR VALUE AT 0.1 SEC.│         └──────────────────┘
│       INTERVALS          │  SENSOR VALUE┌─────────────────┐
└──────────────────────────┘─ ─ ─ ─ ─►│       RAM        │
            │                          └──────────────────┘
            ▼
┌──────────────────────────┐
│PLACEMENT OF FINGER DETECTED?│
└──────────────────────────┘
            │ Y
            ▼              DISPLAYED DATA
┌──────────────────────────┐          ┌──────────────────┐
│    DISPLAY "MEASURING"   │─ ─ ─ ─ ─►│       LCD        │
└──────────────────────────┘          └──────────────────┘
            │
            ▼
           (A)
```

# FIG. 6

SOFTWARE                                              HARDWARE

(A)

PULSE-DRIVE LED OF 810 nm  ┄┄ PULSE ┄┄▶  810nmLED

ACQUIRE EACH TEMPERATURE
AND OPTICAL SENSOR VALUE     ◀┄┄ SENSOR VALUE ┄┄  A/C CONVERTER
AT 0.1 SEC. INTERVALS         ┄┄ SENSOR VALUE ┄┄▶  RAM

N  ◀── 0.5 SEC. ELAPSED?
           │ Y

PULSE-DRIVE LED OF 950 nm  ┄┄ PULSE ┄┄▶  950nmLED

ACQUIRE EACH TEMPERATURE
AND OPTICAL SENSOR VALUE     ◀┄┄ SENSOR VALUE ┄┄  A/D CONVERTER
AT 0.1 SEC. INTERVALS         ┄┄ SENSOR VALUE ┄┄▶  RAM

N  ◀── 0.5 SEC. ELAPSED?
           │ Y

COUNT DOWN THE REMAINING SECONDS  ┄┄ REMAINING SECONDS ┄┄▶  LCD

N  ◀── 10 SEC. ELAPSED SINCE
        START OF MEASUREMENT?
           │ Y

(B)

# FIG. 7

SOFTWARE                                                                                    HARDWARE

(B)

| DISPLAY "PLACE FINGER" | DISPLAYED DATA | LCD |

| ACQUIRE EACH TEMPERATURE SENSOR VALUE AT 0.1 SEC. INTERVALS | SENSOR VALUE | A/D CONVERTER |
| | SENSOR VALUE | RAM |

N — DISENGAGEMENT OF FINGER DETECTED?
                        Y

| CALCULATE PARAMETERS | DISPLAYED DATA | LCD |
| | SUBJECT INFORMATION | EEPROM |
| | REGRESSION FUNCTION PARAMETER MEAN VALUES AND STANDARD DEVIATIONS | EEPROM |
| | REGRESSION FUNCTION PARAMETER MEAN VALUES AND STANDARD DEVIATIONS | RAM |
| | BLOOD Hb CONCENTRATION | EEPROM |
| | BLOOD Hb CONCENTRATION | RAM |

| CALCULATE GLUCOSE CONCENTRATION |

| DISPLAY GLUCOSE CONCENTRATION, AND RECORD IT IN IC CARD, TOGETHER WITH DATE/TIME OF MEASUREMENT | GLUCOSE CONCENTRATION | LCD |
| | GLUCOSE CONCENTRATION, DATE/TIME OF MEASUREMENT | IC CARD |

| | | BUTTON 11b |

| DISPLAY MENU SCREEN | DISPLAYED DATA | LCD |

19

# FIG. 8

SOFTWARE | HARDWARE

| START UP INITIAL PROGRAM | ◄------------------ | POWER SWITCH |

| CHECK CIRCUIT | ◄------------------ | PERIPHERAL CIRCUITS |

MENU

| DISPLAY MENU SCREEN | ------------------► | LCD |

| ◄------------------ | BUTTON 11a |

| SELECT SUBJECT INFORMATION INPUT MODE | ◄------------------ | BUTTON 11b BUTTON 11c |

SELECTED MODE

------------------► | LCD |

| ENTER INTO SUBJECT INFORMATION INPUT MODE | ◄------------------ | BUTTON 11d |

| SELECT "ABLE-BODIED PERSON" OR "DIABETIC PATIENT" AS SUBJECT INFORMATION | ◄------------------ | BUTTON 11b BUTTON 11c |

SUBJECT INFORMATION

------------------► | LCD |

| FINALIZE SUBJECT INFORMATION | ◄------------------ | BUTTON 11d |

SUBJECT INFORMATION

| STORE SUBJECT INFORMATION | ------------------► | EEPROM |

MENU

| DISPLAY MENU SCREEN | ------------------► | LCD |

# FIG. 9

| SOFTWARE | HARDWARE |
|---|---|

| SOFTWARE | | HARDWARE |
|---|---|---|
| START UP INITIAL PROGRAM | ◄------------------- | POWER SWITCH |
| CHECK CIRCUIT | ◄-----------------► | PERIPHERAL CIRCUITS |
| DISPLAY MENU SCREEN | MENU -------------► | LCD |
| | ◄----------------- | BUTTON 11a |
| SELECT THE BLOOD Hb CONCENTRATION INPUT MODE | ◄----------------- | BUTTON 11b BUTTON 11c |
| | SELECTED MODE -----------► | LCD |
| ENTER INTO THE BLOOD Hb CONCENTRATION INPUT MODE | ◄----------------- | BUTTON 11d |
| SET THE BLOOD Hb CONCENTRATION IN BLOOD SAMPLE | ◄----------------- | BUTTON 11b BUTTON 11c BUTTON 11d |
| | SET VALUE -----------► | LCD |
| FINALIZE THE SET VALUE | ◄----------------- | BUTTON 11d |
| STORE THE SET VALUE | SET VALUE -----------► | EEPROM |
| DISPLAY MENU SCREEN | MENU -----------► | LCD |

21

# FIG. 10

```
┌──┐      ┌───┐
│27│ ───▶ │A1 │▷ ───▶ │AD1 │▷ ───▶
└──┘      └───┘
```

CONVERSION OF TEMPERATURE MEASUREMENT DATA INTO NORMALIZED PARAMETERS

CONVERSION OF OPTICAL MEASUREMENT DATA INTO NORMALIZED PARAMETERS

CONVERSION OF NORMALIZED PARAMETERS INTO GLUCOSE CONCENTRATION

28

810nm  950nm

35

A2

A3

AD2

AD3

# FIG. 11

mg / dl

METHOD OF THE INVENTION

600 — 500 — 400 — 300 — 200 — 100

0    100   200   300   400   500   600

mg / dl

COMPARATIVE METHOD
(ENZYMATIC ELECTRODE)

**European Patent Office**

# EUROPEAN SEARCH REPORT

Application Number

EP 04 02 7098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OK KYUNG CHO ET AL: "NONINVASIVE MEASUREMENT OF GLUCOSE BY METABOLIC HEAT CONFORMATION METHOD" CLINICAL CHEMISTRY, AMERICAN ASSOCIATION FOR CLINICAL CHEMISTRY, WASHINGTON, DC, US, vol. 50, no. 10, 12 August 2004 (2004-08-12), XP002357173 ISSN: 0009-9147 Retrieved from the Internet: URL:http://www.clinchem.org/cgi/reprint/cl inchem.2004.036954v1.pdf> [retrieved on 2005-12-01] * the whole document * * figures 1,2,4,6 * | 1-11 | A61B5/00 |
| Y | PATENT ABSTRACTS OF JAPAN vol. 2000, no. 06, 22 September 2000 (2000-09-22) & JP 2000 074829 A (MITSUI CHEMICALS INC), 14 March 2000 (2000-03-14) * abstract * -& JP 2000 074829 A (MITSUI CHEMICALS INC) 14 March 2000 (2000-03-14) | 1-11 | |
| A | US 6 377 828 B1 (CHAIKEN JOSEPH ET AL) 23 April 2002 (2002-04-23) * column 6, lines 8-46 * | 1,5 | |
| A | WO 01/28414 A (KAUFMANN-KIM, YUN-OAK; CHO, OK-KYUNG) 26 April 2001 (2001-04-26) * page 8, lines 10-17 * * page 9, lines 17-24 * | 8,9 | |

TECHNICAL FIELDS SEARCHED (IPC)

A61B

The present search report has been drawn up for all claims

| Place of search | Date of completion of the search | Examiner |
|---|---|---|
| Berlin | 2 December 2005 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 02 7098

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2005

| Patent document cited in search report | | Publication date | Patent family member(s) | | Publication date |
|---|---|---|---|---|---|
| JP 2000074829 | A | 14-03-2000 | NONE | | |
| US 6377828 | B1 | 23-04-2002 | US 6044285 A | | 28-03-2000 |
| WO 0128414 | A | 26-04-2001 | NONE | | |

EPO FORM P0459

For more details about this annex : see Official Journal of the European Patent Office, No. 12/82